# EUROPEAN PATENT APPLICATION

(11) **EP 2 363 069 A1**
(43) Date of publication of application: **07.09.2011**
(21) Application number: 11155150.3
(22) Date of filing: 21.02.2011
(51) Int. Cl.: A61B 6/00

(54) **A phantom**

(30) Priority: 04.03.2010 GB 1003609
(71) Applicant: Leeds Test Objects Limited, Boroughbridge, Yorkshire YO51 9NR (GB)
(72) Inventor: Walker, Adrian, Boroughbridge, Yorkshire YO51 9NR (GB)
(74) Representative: Waddington, Richard

(57) **Abstract**

The invention relates to a phantom for use in the testing of an imaging apparatus. The phantom comprises a body portion; and at least one insert. The or each insert being releasably contained within a recess of the body portion.

## Description

The present invention relates to a testing device for use with a medical imaging apparatus or instrument. In particular, the present invention relates to a phantom for use with a medical imaging apparatus or instrument, to test the calibration and evaluate the performance of the apparatus or instrument. The phantoms are particularly useful in the testing of computed tomography scanners (CT scanners).

In modern imaging undertaken for medical purposes, it is necessary to ensure that images are accurate, clear and well-defined. In order to achieve this, the apparatus and instruments that are used to perform the imaging must be kept in good working order and well-calibrated.

To ensure that the apparatus and instruments are working satisfactorily, it is necessary to test the apparatus and instruments. This testing is done with phantoms. Phantoms are devices which have a set of defined imaging results. Therefore, an image of a specific phantom produced by a particular apparatus or instrument should be within a defined tolerance of the defined result if the apparatus or instrument is working correctly.

Phantoms are usually produced to be instrument specific. However, modern instruments can be used in a number of ways to take images of different parts of a human or animal body. Therefore, phantoms have to be adaptable to allow different settings on an instrument to be tested. This usually involves the phantoms being divided into sections with interchangeable sections for different tests. These section can be confusing and to assemble the phantom is the correct way requires considerable skill and experience.

Due to this aspect of the phantoms, testing of the instruments is usually undertaken by an engineer at an annual or bi-annual frequency. However, the instruments could become faulty, or need re-calibrating, during this period. This would result in inaccurate tests being performed on patients, ultimately leading to potentially wrong diagnoses being made.

It is an object of the present invention to address the above and other disadvantages with the prior art.

According to a first aspect of the present invention, there is provided a phantom for use in the testing of an imaging apparatus; the phantom comprising
a body portion; and
at least one insert,
the or each insert being releasably contained within a recess of the body portion.

According to a further aspect of the present invention, there is provided a kit comprising
a housing for a phantom, the housing comprising at least one recess, and
at least one insert,
the or each insert being adapted to be releasably received within a recess.

Preferably, the body is substantially cylindrical. Preferably, the body is solid.

Preferably, the body comprises a plastics material. Preferably, the body comprises PMMA (polymethyl methacrylate).

Preferably, the body has a first surface and a second surface. Preferably, the first and second surfaces are defined by the circular ends of the cylinder.

Preferably, the body has a longitudinal axis which extends between the first surface and the second surface.

Preferably, the body is open at its first surface and closed at its second surface.

Preferably, the first surface is adapted to receive a closure means.

Preferably, the second surface of the body comprises attachment means. Preferably, the attachment means are adapted to releasably attach the body to a support means. The support means is adapted to support the phantom when in use.

Preferably, the body comprises a plurality of internal cavities. The cavities are preferably enclosed entirely within the body. Preferably, the cavities provide a means of measuring geometric distortion of the imaging apparatus.

Preferably, the body comprises at least one guideline extending along a line parallel to the longitudinal axis of the body. Preferably, the body comprises at least one guideline extending along a line perpendicular to the longitudinal axis of the body

Preferably, the or each guideline is marked on an outer surface of the body. Preferably, the or each guideline is etched into an outer surface of the body.

Preferably, the or each recess is substantially cylindrical. Preferably, the or each recess extends from the first surface of the body towards the second surface of the body.

Preferably, the or each recess has a longitudinal axis which extends along the or each recess in parallel to the longitudinal axis of the body.

Preferably, the body comprises at least two recesses. Preferably, one recess is concentric with the body. Preferably, the body comprises a plurality of recesses.

Preferably, the or each recess is open at the first surface of the body.

Preferably, the or each recess is adapted to receive a closing means at the first surface of the body.

Preferably, the or each recess comprises locating means. Preferably, the or each locating means comprises a groove extending along the circumferential surface of the or each recess in parallel with the longitudinal axis of the or each recess.

Preferably, the or each insert is substantially cylindrical. Preferably, the or each insert has a longitudinal axis. Preferably, the or each insert has a depth along its longitudinal axis of less than its diameter.

Preferably, the or each insert comprises a plastics material, for example PMMA or PTFE.

Preferably, the or each recess is adapted to receive a plurality of inserts.

Preferably, the or each insert is either a testing insert or a blank insert.

Preferably, the or each blank insert is adapted to provide a space between other testing or blank inserts.

Preferably, the or each testing insert is adapted to provide a means of measuring the spatial resolution, contrast and inherent artefacts of the imaging apparatus.

The or each testing insert may be adapted to receive one or more testing objects. The testing objects may be plastics or metal objects of differing densities.

Preferably, the or each insert comprises guide means. Preferably, the or each guide means comprises a ridge which extends along the outer circumferential surface of the insert in parallel with the longitudinal axis of the insert.

Preferably, the or each guide means is adapted to engage with the or each locating means.

Preferably, the or each guideline is adapted to provide a reference for the insertion of the or each insert into the or each recess.

The phantom of the present invention allows test images to be produced by imaging systems for comparison with defined images. The apparatus of the present invention allow routine quality assurance (QA) procedures to be performed by radiographers to ensure the consistent image quality of the imaging system. If measurements from the test images are out of a specified range, the radiographer will know that the imaging system is of non-diagnostic image quality. The imaging system can then be re-calibrated to ensure that it is suitable for diagnosis again.

Any of the above features can be taken in any combination and with any aspect of the invention.

An embodiment of the present invention will now be illustrated further by way of example only, with reference to the accompanying drawings, in which;
Figure 1 shows a schematic, plan view of a phantom housing according to a first embodiment of the invention;
Figure 2 shows a schematic, cross-sectional view through the housing of Figure 1;
Figure 3 shows a schematic, perspective view of the housing of Figure 1 with closing means and securing means;
Figure 4 shows a schematic, perspective view of the housing of Figure 1 showing engraved guidelines;
Figures 5a and b show schematic plan and perspective views of a blank insert to be used with the housing of Figure 1 ;
Figures 6a and b show schematic plan and perspective views of a LSF insert to be used with the housing of Figure 1 ;
Figures 7a to d show schematic plan and perspective views of a PSF insert and securing means to be used with the housing of Figure 1;
Figures 8a to d show schematic plan and perspective views of an artefact insert and securing means to be used with the housing of Figure 1;
Figures 9a to f show schematic plan and perspective views of a spatial resolution (LP/mm) insert, securing means and test object to be used with the housing of Figure 1;
Figures 10a to d show schematic plan and perspective views of another spatial resolution (LP/mm) insert and test object to be used with the housing of Figure 1;
Figures 11 a to d show schematic plan and perspective views of a contrast resolution insert and securing means to be used with the housing of Figure 1; and
Figures 12a to d show schematic plan and perspective views of an HU insert and securing means to be used with the housing of Figure 1.

In the following description, the terms "upper", "top", "above" and "lower", "base" and "below" refer to the device of the present invention as it is presented in the accompanying drawings.

A phantom for use in the testing and calibration of a medical imaging device comprises a housing and a plurality of inserts which can be used with the housing 2 either interchangeably and/or in conjunction with each other. The housing 2 comprises a plurality of recesses. The inserts are adapted to be received within the recesses in the housing 2. In the present example, the phantom is a phantom for the testing and calibration of a CT scanning device.

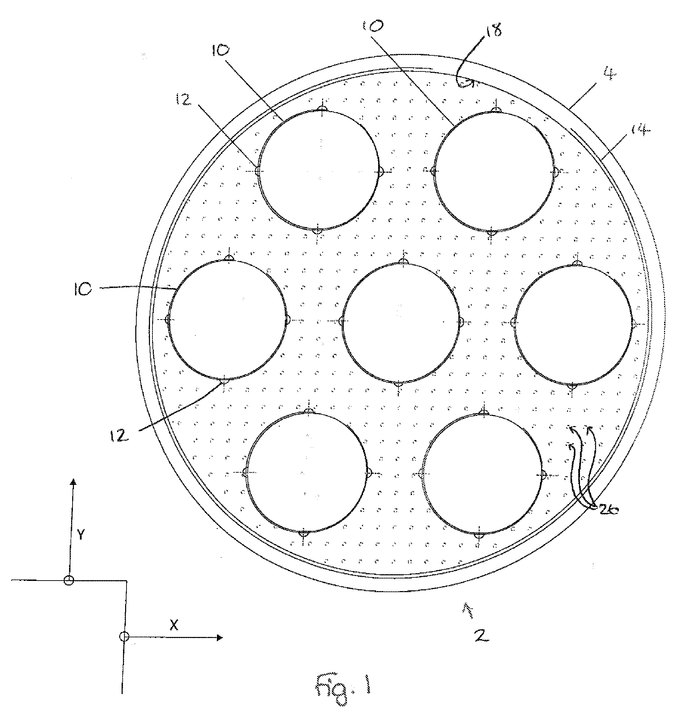

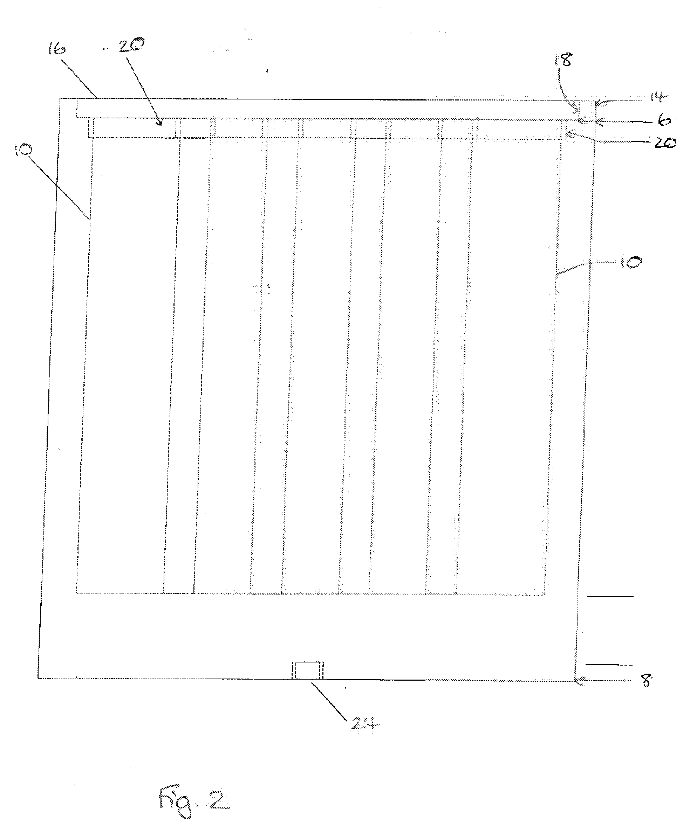

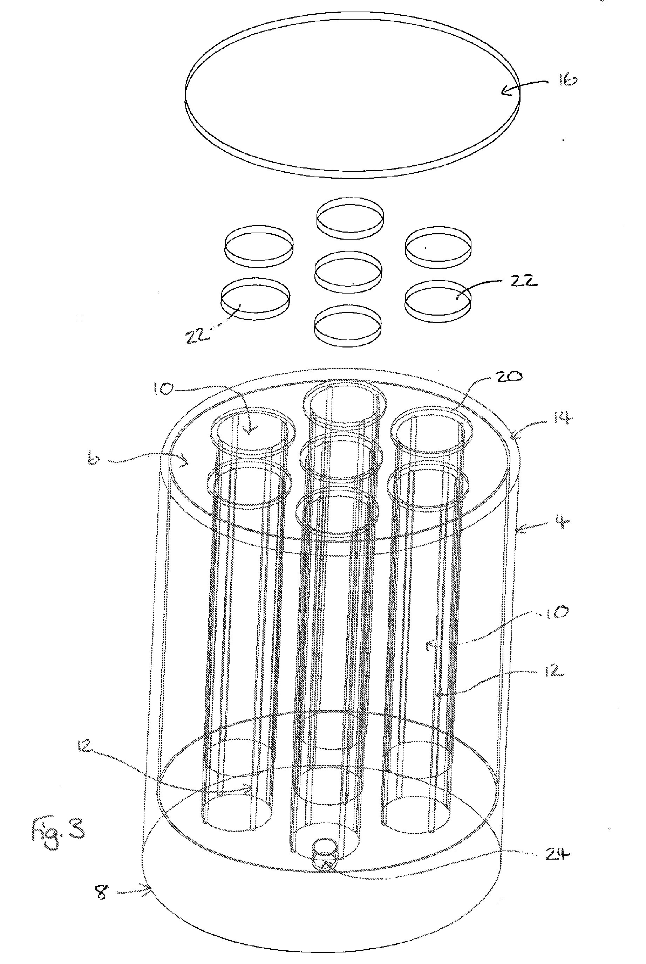

Referring now to Figures 1 to 3, a housing 2 for a phantom comprises a substantially cylindrical body 4. The body 4 has a circular cross-section and a longitudinal axis which extends along the body 4 through the centre of its circular cross-section, i.e. through its centre of rotation. The body 4 has a first surface 6 and a base 8. The first surface 6 is so defined to allow inserts to be placed into the recesses.

The housing 2 is constructed from PMMA (poly(methyl) methacrylate). The housing 2 has a diameter of approximately 160mm, and a depth of approximately 176.7mm.

The housing 2 comprises a plurality of recesses 10. In the present embodiment of the invention, the housing 2 comprises seven recesses 10. The recesses 10 are substantially cylindrical and extend through the body 4 of the housing 2 substantially in parallel to the longitudinal axis of the body 4. The recesses 10 each have the same diameter and each have their own longitudinal axes which extend through the centres of each of the circular cross-sections of the recesses 10 respectively. The recesses 10 extend from the first surface 6 of the body 4 to a point above the base 8 of the body 4. The recesses 10 are open at the first surface 6 of the body 4. The recesses 10 have a diameter of approximately 35.3mm, and a depth of approximately 140mm.

The recesses 10 are arranged within the body 4 of the housing 2 with the longitudinal axis of one recess 10 being co-linear with the longitudinal axis of the body 4, and the remaining six recesses 10 surrounding the centre recess 10 such that the centres of their circular cross-sections form a regular hexagon. The hexagon is located such that each of the vertices, defined by the longitudinal axes of the recesses, are equidistant from the longitudinal axis of the body 4.

The recesses 10 each comprise insert locating means 12. These locating means 12 are adapted to hold the inserts in place within the recesses 10 such that they are unable to rotate. The insert locating means 12 comprise grooves. The grooves 12 extend along the length of each recess, and have a substantially semi-circular cross section. Each recess 10 has four locating means 12. The locating means 12 are evenly spaced around the circumference of the circular cross-section of each recess 10. The diameter of the circular cross-section of each groove 12 is approximately 3mm.

The first surface 6 of the body 4 of the housing 2 comprises a lip 14 to accommodate closure means 16. The lip 14 extends around the circumference of the body 4 and extends upwardly from the first surface 6. The inner surface 18 of the lip 14, i.e. the surface 18 facing inwards towards the longitudinal axis of the body 4, has a threaded surface. The inner surface 18 of the lip 14 has a diameter of approximately 150mm, and is approximately 6mm deep. The threaded surface is adapted to engage with a threaded outer surface on the housing closure means 16.

The housing closure means 16 comprises a disc of PMMA. The disc 16, as mentioned, has a threaded surface on the face pointing radially outwards from the centre of the circular cross-section of the closure means 16. The disc 16 is secured in place on the lip 14 of the body 4 of the housing 2 to keep any inserts within the recesses 10 in the housing 2. The disc 16 is approximately 150mm in diameter and approximately 5mm thick.

A first edge of each of the recesses 10 is surrounded by a cut-out portion 20. Each cut-out portion 20 is concentric with the recess 10 it is surrounding. The cut-out portions 20 have a diameter of approximately 37.3mm and a depth of approximately 5.7mm. The cut-out portions 20 are adapted to receive recess closure means 22. The cut-outs 20 are adapted to engage with the recess closure means 22 in an analogous way to the way in which the lip 14 engages with the housing closure means 16. The faces of the cut-outs 20 which face the longitudinal axes of the recesses 10 are threaded, and adapted to engage threaded surfaces of the recess closure means 22.

The recess closure means 22 comprise small discs of PMMA. The discs 22 have a diameter of approximately 37.3mm, and are 5mm thick.

The base of the housing 2 comprises a small recess 24. The internal surface of the recess 24 is threaded. The threaded recess 24 is adapted to be releasably secured to a support means (not shown), for example a tripod or table, having a correspondingly threaded protrusion. The support means allow the phantom to be held in a suitable configuration within an imaging device (not shown) to allow an image of the phantom to be produced by the imaging device.

As shown in Figure 1, the body 4 of the phantom comprises a plurality of internal cavities 26. The cavities 26 are substantially cylindrical with a cross-sectional diameter of approximately 1 mm and a depth of approximately 3mm. The cavities 26 are located within the body 4 of the phantom, substantially in a plane which extends across the circular cross-section of the body 4 at approximately half the height of the body 4. The cavities 26 are an imaging tool. When an image of the phantom is produced by an imaging system, the cavities 26 provide a result which can be used to double check for any geometric distortion in the produced image.

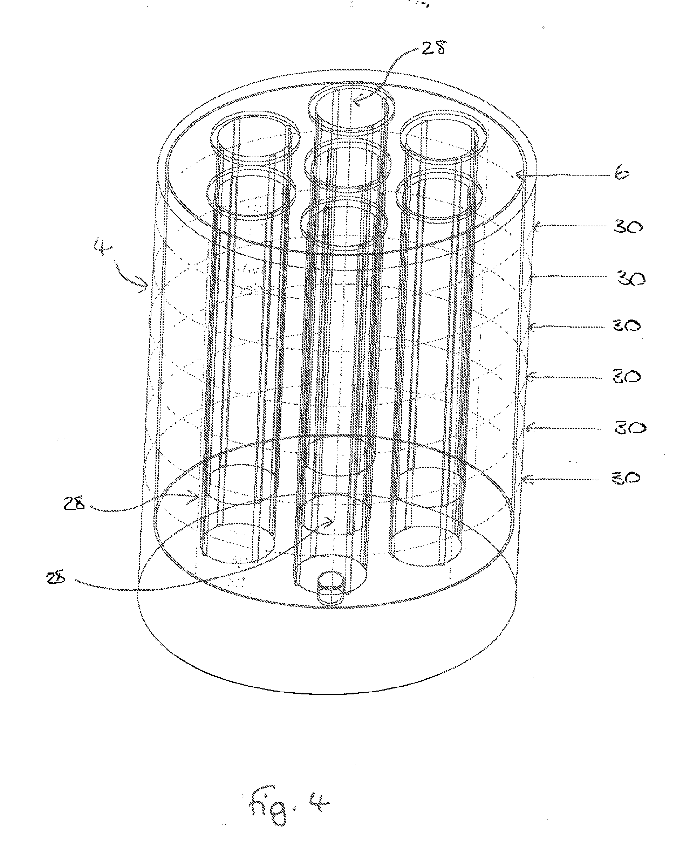

Referring now to Figure 4, the housing 2 comprises a series of guidelines 28,30. The guidelines 28,30 assist in the positioning of testing inserts within the recesses 10. The guidelines 28,30 comprise a series of lines that are etched into the body 4 of the housing 2. The lines are etched in lines which are parallel 28 to the longitudinal axis of the body 4 and perpendicular 30 to the longitudinal axis of the body 4. The lines 28 which are parallel to the longitudinal axis of the body 4 are arranged in radial alignment with the longitudinal axes of the six outer recesses 10. The lines 30 which are perpendicular to the longitudinal axis of the body 4 extend circumferentially around the outer surface of the body 4. The circumferential lines 30 start at approximately 20mm below the first surface 6 of the housing 2 and are then spaced at 20mm intervals along the body 4 to the depth of the recesses 10. There are seven circumferential lines 30 extending in total approximately 140mm along the body 4.

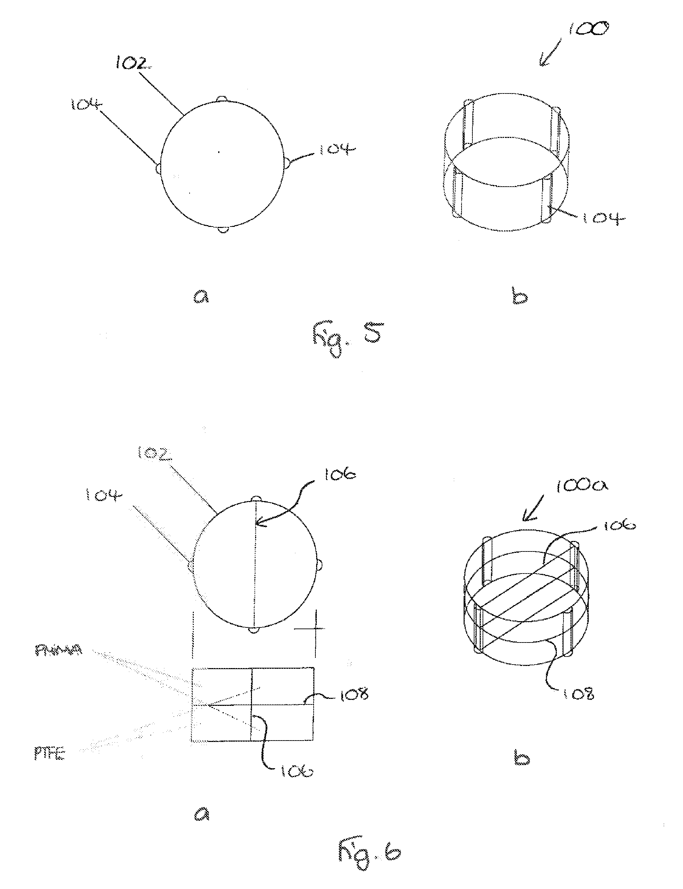

Referring now to Figures 5 to 12, inserts 100 for the phantom are shown and described. The inserts 100 are adapted to be received within the recesses 10 in the housing 2, and kept in place by the recess closure means 22 and housing closure means 16. The inserts 100 are adapted to provide defined imaging patterns when scanned by an accurate CT scanner at different points within the phantom. Therefore, the testing inserts 100 can be used to check the accuracy and calibration of the imaging device by comparing the actual image produced by the CT scanner with a defined image.

Figures 5a and b show plan and perspective views respectively of a blank insert 100. The blank inserts 100 are used to put spaces between the testing inserts that will be described below in more detail in relation to Figures 6 to 12.

The blank insert 100 comprises a substantially cylindrical block 102 of PMMA. The insert 100 has a diameter to correspond with the diameter of the recesses 10, to allow the insert 100 to be placed easily within the recesses 10 in the body 4 of the phantom. The insert 100 has a diameter of approximately 34.5mm (±0.25mm), and is approximately 20mm deep.

The blank insert 100 comprises a number of guide members 104. The guide members 104 comprise ridges which extend along the depth of the insert 100 in parallel configuration to the longitudinal axis of the cylinder. The guide members 104 are adapted to engage with the insert locating means 12 in the recesses 10. Therefore, the ridges 104 have a substantially semi-circular cross-section of approximate diameter 2.5mm.

Figures 6 to 12 show examples of different testing inserts 100a-g which can be used in conjunction with the phantom body 4 described above. The testing inserts 100a-g have substantially the same overall dimensions as the blank insert 100 described above with reference to Figure 5, and the same guide members 104 as those described in relation to Figure 5. In the following figures, like reference numerals will be used to denote like features of the inserts.

Figures 6a and b show plan and perspective views respectively of a line spread function (LSF) testing insert 100a. The LSF testing insert 100a comprises a cylindrical block 102 divided into quarters by two intersecting planes. The first plane 106 extends across the diameter of the block from a first surface of the block to a second surface of the block 102. The second plane 108 extends across the circular cross-section of the block 102 at approximately half way between the first and second surfaces of the block 102.

The block 102 is formed from two separate materials, PMMA and PTFE (polytetrafluoroethylene). The block 102 is constructed such that adjacent quarters comprise different materials. Therefore, when viewed from the side along the plane 106 extending across the diameter of the block 102, each PMMA quarter is adjacent to two PTFE quarters, and each PTFE quarter is adjacent to two PMMA quarters.

The guide members 104 are formed from PMMA similarly to those described with reference to Figure 5.

The LSF testing insert 100a is adapted to provide a measure of spatial resolution in am image thereof produced by an imaging system. In particular, the LSF testing insert 100a is adapted to allow the ability of the imaging system to accurately reproduce a high:low contrast straight edge to be measured.

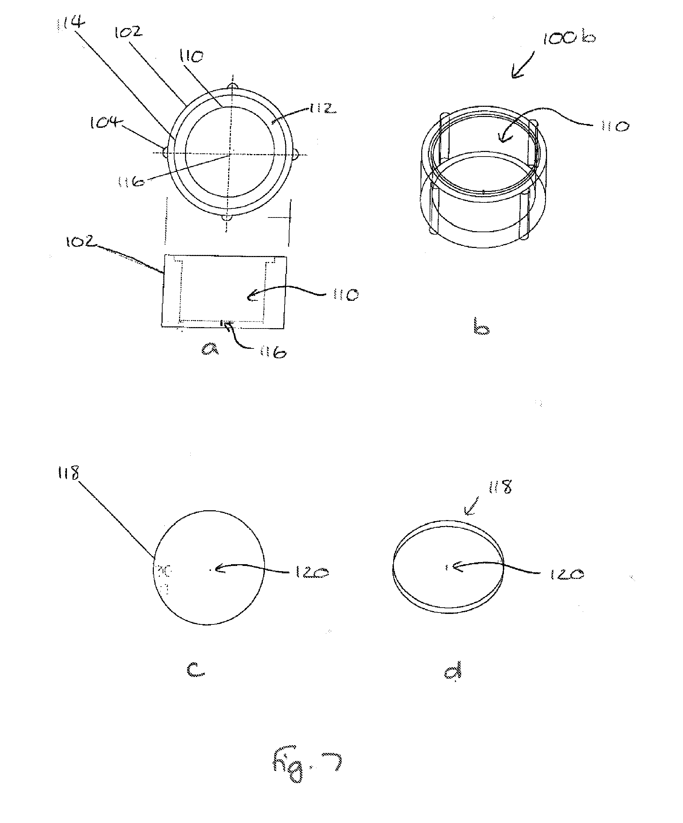

Referring now to Figures 7 a and b, plan and perspective views respectively of a point spread function (PSF) testing insert 100b are shown. The PSF testing insert 100b comprises a cylindrical block 102 of PMMA having a central recess 110.

The recess 110 has a diameter of approximately 25mm and is concentric with the circumference of the block 102. The recess 110 extends to approximately 1.5mm above the second surface 128 of the insert 100b. The first surface 112 of the insert 100b comprises a lip 114 which is approximately 1.5mm deep and has an internal diameter of approximately 31.5mm. The internal circumference of the lip 114 is concentric with the circumference of the block 102. The internal circumference of the lip 114 is threaded.

In the centre of the circular base of the recess 110, there is a notch 116 of approximately 0.25mm in diameter and approximately 1mm in depth which extends towards the second surface 128 of the block 102.

The first surface 112 of the block 102 is closed by a closure disc 118 with a threaded outer surface corresponding to the internal circumference of the lip 114 as shown in Figures 7c and d. The closure disc 118 has a notch 120 of the same dimensions as the notch 116 in the base of the recess 110 in a corresponding location to that of the base of the recess 110, i.e. the notch 120 is approximately 0.25mm in diameter and approximately 1 mm in depth and extends towards the first surface of the block.

The PSF testing insert 100b is adapted to provide a measure of spatial resolution in am image thereof produced by an imaging system. In particular, the PSF testing insert 100b is adapted to allow the ability of the imaging system to accurately reproduce a high contrast 'dot' surrounded by a low contrast medium to be measured.

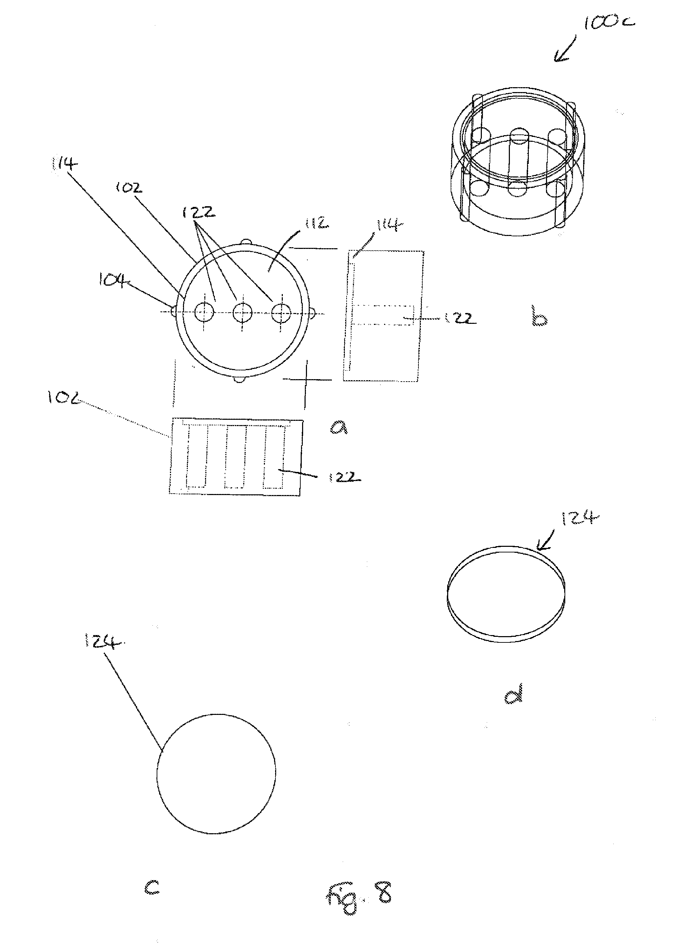

Referring now to Figures 8a and b, plan and perspective views of an artefact testing insert 100c are shown. The artefact testing insert 100c comprises a substantially cylindrical block 102 of PMMA having three recesses 122 extending from a first surface 112 towards a second surface 128 of the block 102.

The recesses 122 are arranged in a line extending across a diameter of the block 102. The recesses 122 are substantially cylindrical in shape with a diameter of approximately 5.15mm and a depth of approximately 17mm. The recesses 122 are adapted to receive either cylinders of titanium or lead.

The first surface 112 of the insert 100c comprises a lip 114 which is approximately 1.5mm deep and has an internal diameter of approximately 31.5mm. The internal circumference of the lip 114 is concentric with the circumference of the block. The internal circumference of the lip 114 is threaded.

The first surface 112 of the block 102 is closed by a closure disc 124 with a threaded outer surface corresponding to the internal circumference of the lip 114 as shown in Figures 8c and d. The closure disc 124 comprises a disc of PMMA. The closure disc 124 has a diameter of approximately 31.5mm and a depth of approximately 1.5mm.

The artefact testing insert 100c is adapted to create imaging artefacts inherent in the imaging process which can then be measured.

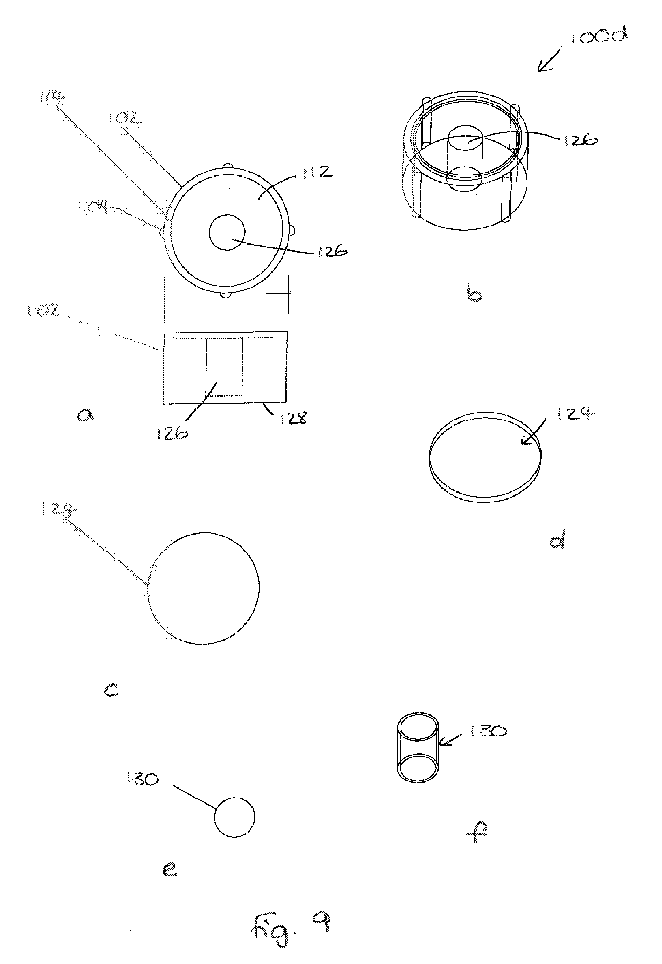

Referring to Figures 9a and b, plan and perspective views of a Z line pairs per millimetre spatial resolution (LP/mm) testing insert 100d are shown. The Z LP/mm testing insert 100d comprises a substantially cylindrical block 102 of PMMA having a central recess 126.

The recess 126 has a diameter of approximately 11.1 mm and depth of approximately 17mm, and is concentric with the circumference of the block 102. The recess 126 extends to approximately 1.5mm above the second surface 128 of the insert 100d. The first surface 112 of the insert 100d comprises a lip 114 which is approximately 1.5mm deep and has an internal diameter of approximately 31.5mm. The internal circumference of the lip 114 is concentric with the circumference of the block 102. The internal circumference of the lip 114 is threaded.

The first surface 112 of the block 102 is closed by a closure disc 124 with a threaded outer surface corresponding to the internal circumference of the lip 114 as shown in Figures 9c and d. The closure disc 124 comprises a disc of PMMA. The closure disc 124 has a diameter of approximately 31.5mm and a depth of approximately 1.5mm.

The Z LP/mm testing insert 100d further comprises a testing object 130, as shown in Figures 9e and f. The testing object 130 comprises a hollow cylinder of PMMA. The object 130 has an outer diameter of 11.1 mm, and is adapted to fit within the central recess 126 in the insert 100d.

The Z LP/mm testing insert 100d is adapted to provide a measure of spatial resolution in an image thereof produced by an imaging system. In particular, the Z LP/mm testing insert 100d is adapted to allow the ability of the imaging system to resolve alternating low and high contrast bars of increasingly fine spatial resolution in the Z plane to be measured.

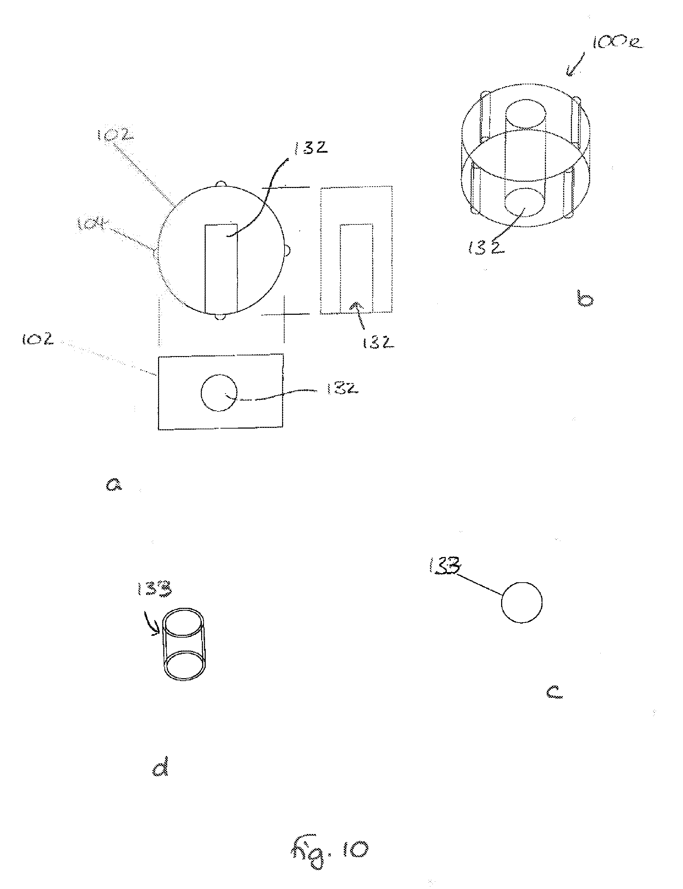

Referring to Figures 10a and b, plan and perspective views of an X-Y line pairs per millimetre spatial resolution (LP/mm) testing insert 100e are shown. The X-Y LP/mm testing insert 100e comprises a substantially cylindrical block 102 of PMMA having a diametrical recess 132.

The recess 132 extends into the block 102 from a circumferential surface of the block 102 in a diametrical direction. The recess 132 has a diameter of approximately 11.1 mm and depth of approximately 24mm.

The X-Y LP/mm testing insert 100e further comprises a testing object 133, as shown in Figures 10c and d. The testing object 133 comprises a hollow cylinder of PMMA. The object 133 has an outer diameter of 11.1 mm, and is adapted to fit within the diametrical recess 132 in the insert 100e.

The X-Y LP/mm testing insert 100e is adapted to provide a measure of spatial resolution in an image thereof produced by an imaging system. In particular, the X-Y LP/mm testing insert 100e is adapted to allow the ability of the imaging system to resolve alternating low and high contrast bars of increasingly fine spatial resolution in the X-Y plane to be measured.

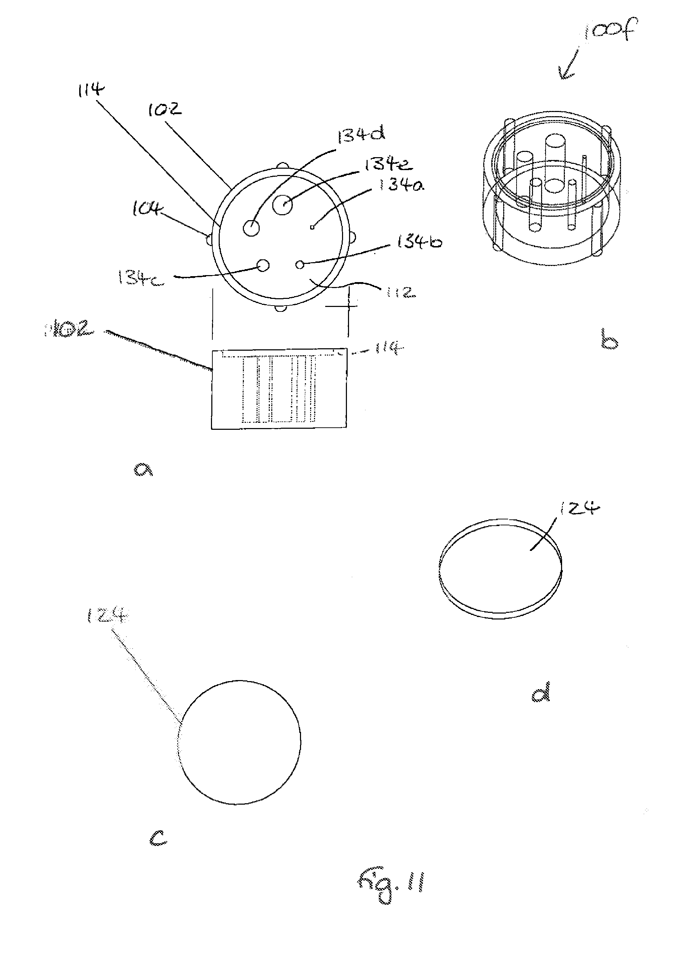

Referring now to Figures 11 a and b, plan and perspective views of a contrast resolution insert 100f are shown. The contrast resolution insert 100f comprises a substantially cylindrical block 102 of PMMA having a plurality of recesses 134a-e.

In the example shown in Figure 11, the insert 100f comprises five recesses. The recesses 134a-e each have a depth of 17mm. The recesses have diameters of 5mm (134e), 4mm (134d), 3mm (134c), 2mm (134b) and 1 mm (134a) and are arranged such that each recess is centred on a corner of a regular pentagon with sides of 10mm, the pentagon being centred in the cylindrical block 102.

The first surface 112 of the insert 100f comprises a lip 114 which is approximately 1.5mm deep and has an internal diameter of approximately 31.5mm. The internal circumference of the lip 114 is concentric with the circumference of the block 102. The internal circumference of the lip 114 is threaded.

The first surface 112 of the block 102 is closed by a closure disc 124 with a threaded outer surface corresponding to the internal circumference of the lip 114 as shown in Figures 11c and d. The closure disc 124 comprises a disc of PMMA. The closure disc 124 has a diameter of approximately 31.5mm and a depth of approximately 1.5mm.

The contrast resolution testing insert 100f creates a series of discs of a range of diameters (1mm to 5mm) and different contrasts with the background PMMA. The threshold of detectability of these discs can be measured.

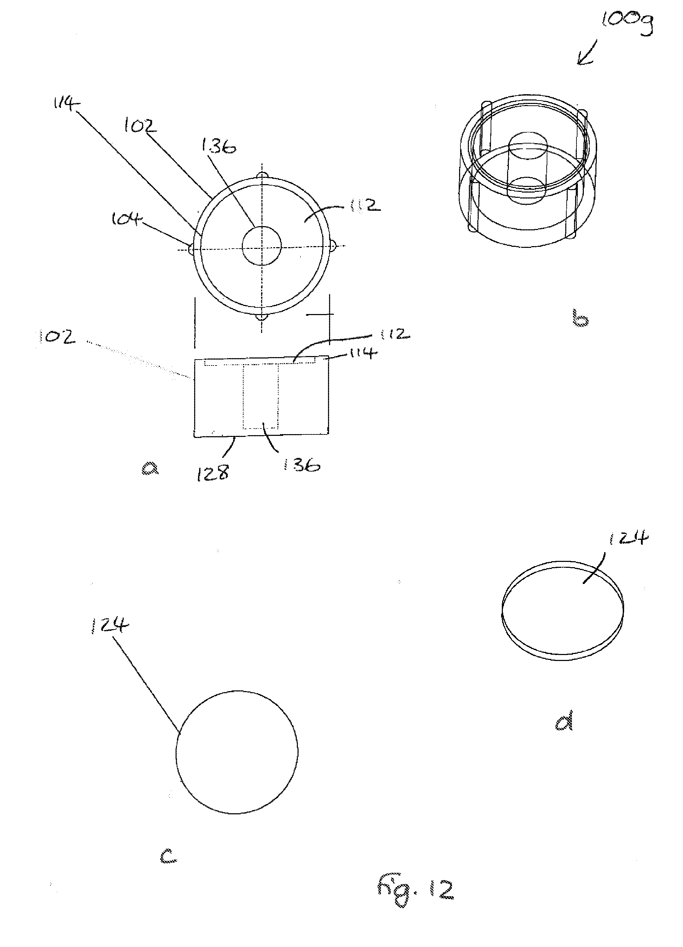

Referring to Figures 12a and b, plan and perspective views of a Hounsfield Unit (HU) testing insert 100g are shown. The HU testing insert 100g comprises a substantially cylindrical block 102 of PMMA having a central recess 136.

The recess 136 has a diameter of approximately 11.1 mm and depth of approximately 17mm, and is concentric with the circumference of the block 102. The recess 136 extends to approximately 1.5mm above the second surface 128 of the insert 100g.

The first surface 112 of the insert 100g comprises a lip 114 which is approximately 1.5mm deep and has an internal diameter of approximately 31.5mm. The internal circumference of the lip 114 is concentric with the circumference of the block 102. The internal circumference of the lip 114 is threaded.

The first surface 112 of the block 102 is closed by a closure disc 124 with a threaded outer surface corresponding to the internal circumference of the lip 114 as shown in Figures 12c and d. The closure disc 124 comprises a disc of PMMA. The closure disc 124 has a diameter of approximately 31.5mm and a depth of approximately 1.5mm.

The HU testing insert 100g further comprises a number of testing objects (not shown) of differing materials, which are adapted to be inserted alternatively into the central recess 136.

The HU testing insert 100g creates a series of regions of interest with a range of contrasts of known HU. The measured HU value for each region in an image of the testing insert 100g produced by an imaging system should be within a specified tolerance of the known value.

In use, the or each insert 100,100a-g, as desired, is placed into a recess 10 in the body 4 of the housing 2 such that the guide members 104 engage with the locating means 12. Once the desired inserts 100,100a-g have been placed in the desired locations within the housing 2, the recess closure means 22 are secured in place. The housing closure means 16 is then secured in place on the first surface 6 of the body 4.

Once the phantom has been securely assembled, as described above, it is inserted into the CT scanner. The CT scanner produces an image of the phantom from which measurements are taken. The scanner is deemed to be correctly calibrated if those measurements are within the specified tolerances. If the measurements are outside the specified range, the user can calibrate the CT scanner so that accurate images are produced.

The device of the present invention provides a phantom which enables the required testing configuration of inserts to be easily and simply created by all users of medical imaging apparatus. This means that calibration of imaging apparatus can be performed by the users of the apparatus instead of engineers being required to undertake the testing.

By ensuring that all users of the imaging apparatus can test and calibrate the apparatus, medical practitioners are able to ensure that the images produced by the apparatus are accurate, and can make a better diagnosis. Furthermore, allowing more users to test and calibrate apparatus will reduce the length of time that apparatus is unusable because an engineer can not be found to test and calibrate the apparatus.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A phantom for use in the testing of an imaging apparatus; the phantom comprising
a body portion; and
at least one insert,
the or each insert being releasably contained within a recess of the body portion.

2. A phantom as claimed in claim 1, wherein the body is substantially cylindrical, comprising a first surface and a second surface, and a longitudinal axis which extends between the first surface and the second surface.

3. A phantom as claimed in claim 2, wherein the second surface of the body comprises attachment means, the attachment means being adapted to releasably attach the body to a support means.

4. A phantom as claimed in any one of the preceding claims, wherein the body comprises a plurality of internal cavities.

5. A phantom as claimed in claim 4, wherein the cavities are enclosed entirely within the body, the cavities being operable to provide a means of measuring geometric distortion of the imaging apparatus.

6. A phantom as claimed in any one of the preceding claims, wherein the body comprises at least one guideline extending along a line parallel to the longitudinal axis of the body.

7. A phantom as claimed in any one of the preceding claims, wherein the or each recess extends from the first surface of the body towards the second surface of the body.

8. A phantom as claimed in claim 7, wherein the or each recess comprises locating means.

9. A phantom as claimed in claim 8, wherein the or each locating means comprises a groove extending along the circumferential surface of the or each recess in parallel with a longitudinal axis of the or each recess.

10. A phantom as claimed in any one of the preceding claims wherein the or each insert is substantially cylindrical, having a longitudinal axis, the or each recess being adapted to receive a plurality of inserts.

11. A phantom as claimed in any one of the preceding claims wherein the or each insert comprises a plastics material, for example PMMA or PTFE.

12. A phantom as claimed in any one of the preceding claims wherein the or each insert is either a testing insert or a blank insert.

13. A phantom as claimed in claim 12, wherein the or each testing insert is adapted to provide a means of measuring the spatial resolution, contrast and inherent artefacts of the imaging apparatus.

14. A phantom as claimed in any one of the preceding claims wherein the or each insert comprises guide means.

15. A kit comprising
a housing for a phantom, the housing comprising at least one recess, and
at least one insert,
the or each insert being adapted to be releasably received within a recess.
